# EUROPEAN PATENT APPLICATION

(11) **EP 3 225 157 A1**
(43) Date of publication of application: **04.10.2017**
(21) Application number: 16305364.8
(22) Date of filing: 30.03.2016
(51) Int. Cl.: A61B 5/00

(54) **SYSTEM COMPRISING A WEARABLE DEVICE AND A WEARABLE SENSOR**

(71) Applicant: Essilor International, 94220 Charenton Le Pont (FR)
(72) Inventor: ROUSSEAU, Denis, 94227 CHARENTON-LE-PONT CEDEX (FR); LORE, Marie, 94227 CHARENTON-LE-PONT CEDEX (FR)
(74) Representative: Cabinet Novitech

(57) **Abstract**

A system comprising a wearable device and a wearable sensor, each one being configured to be in communication with the other one when the wearable device and the wearable sensor are in contact, the wearable sensor when worn by a wearer is configured to measure data relative to the wearer, and the wearable device comprises at least:
- a communication component configured to receive data from the wearable sensor when the wearable sensor is in contact with the wearable device, and
- a power source for providing electrical power to the wearable sensor when the wearable sensor is in contact with the wearable device.

## Description

### FIELD OF THE INVENTION

The invention relates to a system comprising a wearable device and a wearable sensor and a method for communicating between said wearable device and said wearable sensor.

The invention further relates to a computer program product comprising one or more stored sequences of instructions that are accessible to a processor and which, when executed by the processor, causes the processor to carry out the steps of the method according to the invention.

### BACKGROUND OF THE INVENTION

Small wearable sensors, such as skin patches, are now easy to manufacture thanks to plastic electronic technology progress. It is now possible to embed sensors into a very thin and transparent plastic layer that can be directly stuck on the skin.

However, as such sensors are very thin, they cannot embed directly all functions necessary to make an autonomous system, and so they need to be able to communicate with another device.

These existing sensors can make measurements in an autonomous way, but need to be connected to a specific reader, like a mobile phone or RFID reader, to communicate the information stored on the local patch.

There is no possibility to communicate in real time over a long period, because the communication device is a special device that cannot be placed permanently close to the patch.

Therefore, there is a need for a solution allowing to measure in an autonomous way, in real time and over a long period of time using a wearable sensor.

An aim of the present invention is to propose such solution.

### SUMMARY OF THE INVENTION

To this end, the invention proposes a system comprising a wearable device and a wearable sensor, each one being configured to be in communication with the other one when the wearable device and the wearable sensor are in contact, the wearable sensor when worn by a wearer is configured to measure data relative to the wearer, and the wearable device comprises at least:
- a communication component configured to receive data from the wearable sensor when the wearable sensor is in contact with the wearable device, and
- a power source for providing electrical power to the wearable sensor when the wearable sensor is in contact with the wearable device.

Advantageously, the system according to the invention comprising a wearable device and wearable sensor in contact with one another allows measuring data relative to a wearer using a wearable sensor in an autonomous way, in real time and over a long period of time using a wearable sensor. The wearable device may comprise a larger battery and processing means than the wearable sensor, facilitating battery charging and data processing.

According to further embodiments which can be considered alone or in combination:
- the wearable device is a wristband and/or a watch and/or a head mounted device; and/or
- the wearable sensor is a sensor configured to be attached to the body of the wearer; and/or
- the wearable sensor is a patch configured to be glued on the body of the wearer and/or an implant comprising a connector configured to be glued on the body of the wearer and/or a piercing configured to be attached to the body of the wearer; and/or
- the wearable device and the wearable sensor are connected with a removable wire; and/or
- the wearable device and the wearable sensor comprise each at least two connecting plots configured to be in contact when the wearable device and the wearable sensor are in communication; and/or
- each of the wearable device and of the wearable sensor comprises a plurality of connecting plots and wherein the computer executable instructions comprises instructions for determining for at least two connecting plots of the wearable device, two corresponding connecting plots of the wearable sensor each matching with a connecting plot of the wearable device; and/or
- the wearable device comprises a memory configured to store data received from the wearable sensor; and/or
- the communication component of the wearable device is configured to transmit data received from the wearable sensor to a distant unity comprising a storage unit.

The invention further relates to a method for communicating between a wearable device and a wearable sensor, the wearable sensor being configured to be worn by a wearer and to measure data relative to the wearer when worn by the wearer, the wearable device comprising a communication component and a power source, the method comprising at least when the wearable device and the wearable sensor are in contact:
- a data receiving step, during which data is received by the communication component from the wearable sensor, and
- an electrical power providing step, during which electrical power is provided to the wearable sensor from the power source.

According to further embodiments which can be considered alone or in combination:
- the method of the invention may further comprise a data storing step, during which data received from the wearable sensor are stored in a memory embedded in the wearable device; and/or
- each of the wearable device and of the wearable sensor comprises a plurality of connecting plots and wherein the method further comprises an optimal couple of connecting plots determining step, during which for at least two connecting plots of the wearable device, at least two corresponding connecting plots of the wearable sensor each matching with a connecting plot of the wearable device are determined; and/or
- the method of the invention may further comprising a data transmitting step, during which data received from the wearable sensor are transmitted from the communication component of the wearable device to a distant unity comprising a storage unit.

The invention further relates to a computer program product comprising one or more stored sequences of instructions that are accessible to a processor and which, when executed by the processor, causes the processor to carry out at least the data collecting step and the personalizing step of the method according to the invention.

The invention also relates to a computer-readable storage medium having a program recorded thereon; where the program makes the computer execute at least the data collecting step and the personalizing step of the method of the invention.

The invention further relates to a device comprising a processor adapted to store one or more sequence of instructions and to carry out at least the data collecting step and the personalizing step of the method according to the invention.

Unless specifically stated otherwise, as apparent from the following discussions, it is appreciated that throughout the specification discussions utilizing terms such as "computing", "calculating", or the like, refer to the action and/or processes of a computer or computing system, or similar electronic computing device, that manipulate and/or transform data represented as physical, such as electronic, quantities within the computing system's registers and/or memories into other data similarly represented as physical quantities within the computing system's memories, registers or other such information storage, transmission or display devices.

Embodiments of the present invention may include apparatuses for performing the operations herein. This apparatus may be specially constructed for the desired purposes, or it may comprise a general purpose computer or Digital Signal Processor ("DSP") selectively activated or reconfigured by a computer program stored in the computer. Such a computer program may be stored in a computer readable storage medium, such as, but is not limited to, any type of disk including floppy disks, optical disks, CD-ROMs, magnetic-optical disks, read-only memories (ROMs), random access memories (RAMs) electrically programmable read-only memories (EPROMs), electrically erasable and programmable read only memories (EEPROMs), magnetic or optical cards, or any other type of media suitable for storing electronic instructions, and capable of being coupled to a computer system bus.

The processes presented herein are not inherently related to any particular computer or other apparatus. Various general purpose systems may be used with programs in accordance with the teachings herein, or it may prove convenient to construct a more specialized apparatus to perform the desired method.

The desired structure for a variety of these systems will appear from the description below. In addition, embodiments of the present invention are not described with reference to any particular programming language. It will be appreciated that a variety of programming languages may be used to implement the teachings of the inventions as described herein.

### BRIEF DESCRIPTION OF THE DRAWINGS

Embodiments of the invention will now be described, by way of example only, and with reference to the following drawings in which:
- Figure 1 is a schematic representation of a system according to the invention, and
- Figure 2 is flow chart of a method according to the invention.

### DETAILED DESCRIPTION OF PREFERRED EMBODIMENTS

Elements in the figures are illustrated for simplicity and clarity and have not necessarily been drawn to scale. For example, the dimensions of some of the elements in the figure may be exaggerated relative to other elements to help improve the understanding of the embodiments of the present invention.

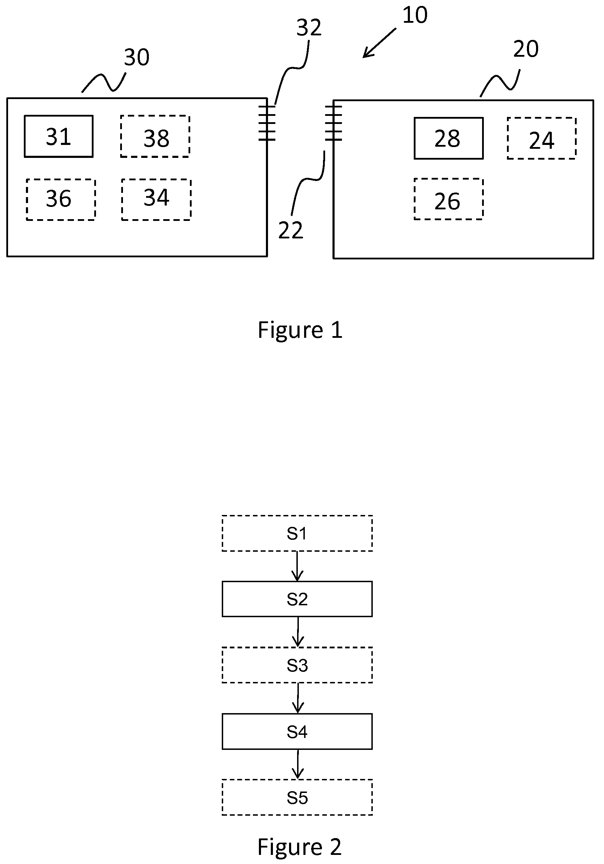

Figure 1 represents an example of a system 10 according to the invention. As illustrated on figure 1, the system 10 comprises a wearable device 20 and a wearable sensor 30.

The wearable device 20 and the wearable sensor 30 comprise contact points 22 and 32 respectively so as to allow an electrical contact between the wearable device and the wearable sensor.

The wearable device 20 and the wearable sensor 30 are configured to be in communication with one another when the wearable device and the wearable sensor are in contact.

As illustrated on figure 1, the wearable device comprises at least a communication component 24 and a power source 26.

The communication component 24 is configured to receive data from the wearable sensor 30 when the wearable sensor is in contact with the wearable device 20.

The power source 26 provides electrical power to the wearable device 20 and to the wearable sensor when the wearable sensor is in contact with the wearable device. For example, the power source 26 may comprise a battery.

The battery may be a Lithium Ion type battery or any other technology like super-capacitor or similar technology allowing storing electrical energy.

The wearable device 20 may comprise charging means adapted to charge the power source 26, for example using a wire or a wireless charging device.

The wearable device is typically a device than may be worn over the day or days and is easily removed, such as a wristband and/or a watch and/or a head mounted device, such as a smart frame or spectacle frame.

Advantageously, the wearable device may comprise a large battery and communication component to facilitate local battery charging and data communication.

The wearable device when in contact with the wearable sensor may retrieve data from the sensor and provide electrical power to the wearable sensor.

The communication component 24 may further be configured to transmit, for example send, data to a distant unit comprising a storage unit. The data sent to the distant entity may be directly the data received from the wearable sensor or may be data generated based on the data received from the wearable sensor.

The distant unit may be a personal digital assistant, an audio/video device, a mobile phone, a MPEG-1 Audio Layer 3 (MP3) player, a personal computer, a laptop, a tablet, a bluetooth headset, a watch, a wristband, etc...

Such distant unit may typically communicate with the communication component 24 of the wearable device 20 by wireless communication. The wireless communication can use different communication protocols such a Bluetooth, Zigbee, Wifi or others.

The wearable device 20 may further comprise a processing unit 28 comprising a processor and a memory so as to store and process the data received from the wearable sensor 30. The memory may be arranged to store computer executable instructions that may be executed by the processor.

The wearable sensor 30 comprises at least one sensing unit 31 configured to sense at least one parameter relative to the wearer of the wearable sensor. In other words, the wearable sensor is configured to measure data relative to the wearer of the wearable sensor when worn by said wearer.

The wearable sensor may be configured to be attached to the body of the wearer.

For example, the wearable sensor may be a patch configured to be glued on the body of the wearer, such as a skin patch.

The wearable sensor may further be an implant sensor, implanted under the skin of the wearer, and comprising a connector configured to be glued on the body of the wearer, for example a skin patch connector. Such sensor may be blood sugar or blood pressure monitoring sensors.

The wearable sensor may be a piercing configured to be attached to the body of the wearer, for example the nose or ear of the wearer.

The wearable sensor may further comprise a battery 36 to store and provide electrical power to the sensors. For example, the wearable sensor may comprise a flexible thin battery that may be recharge when the wearable sensor is in contact with the wearable device.

The wearable sensor may further comprise a processing unit 38 comprising a processor and a memory so as to store and process the sense data prior to sending them to the wearable device.

The wearable sensor may also comprise a communication unit 36 arranged to communicate with the communication component 24 of the wearable device 20.

As indicated previously, the wearable device 20 and the wearable sensor 30 are arranged to communicate with one another when in contact.

According to an embodiment of the invention, the wearable device and the wearable sensors are connected with at least one removable wire. The removable wire offers a power and data connection between the wearable sensor and device.

The wearable device and the wearable sensor may comprise each at least two connecting plots configured to be in contact when the wearable device and the wearable sensor are in communication.

The relative positioning of the wearable device and the wearable sensor may not be accurate and they may move relative one to the other during the day, therefore the connecting plots may be adapted to help assure contact between the wearable device and the wearable sensor.

For example, the connecting plots may comprise magnets to enable easy and painless connection between the wearable device and the wearable sensor.

According to an embodiment of the invention, the wearable device and sensor comprise a plurality of connecting plots and the invention comprises a dynamic search of the best couples of connecting plots to match to transmit power and data between the wearable device and the sensor.

For example, the computer executable instructions stored in the memory of the wearable device may comprise instructions for determining for at least two connecting plots of the wearable device, two corresponding connecting plots of the wearable sensor each matching with a connecting plot of the wearable device.

The wearable sensor may also comprise a memory storing computer executable instructions for determining for at least two connecting plots of the wearable device, two corresponding connecting plots of the wearable sensor each matching with a connecting plot of the wearable device.

For example, when the wearable device is a wristband or a watch and the wearable sensor is a connected patch; both the wristband and the patch may comprise a plurality of connecting plots. A software application may select the connecting plots of the wristband that are in touch with the connecting plots of the patch, depending on how the wristband is positioned.

For example, when the wearable device is a wristband or a watch and the wearable sensor is a connected implant and if the implant is not close to the wristband, a small connector can be stick at the implant place and allow the communication. The connector may be a small flat cable coming from the wristband, and can be glued or magnetically positioned on the implant. Magnets allow a good positioning even if the implant is not visible both the wristband and the patch may comprise a plurality of connecting plots.

For example, when the wearable device is a spectacle frame or smart frame and the wearable sensor is a connected patch; it is possible to have connecting points where the frame is in touch with the wearer face, for example around the ears and nose of the wearer. These connecting points should be large enough to allow a good connection even if the frame moves from the initial position.

When the wearable sensor is an implant and the wearable device a spectacle frame or smart frame, a flex connector from the frame can be used to place the contact points very close to the implant.

Although the invention has been described with one wearable sensor and one wearable device, the system of the invention may comprise a plurality of wearable sensors communicating with one or a plurality of wearable devices.

For example, the system may comprise a first sensor at the wrist of the wearer communicating with a wristband or a watch and a second sensor around the ears of the wearer communicating with a smart frame.

The system may also comprise a plurality of wearable sensors communicating with a single wearable device. For example, the system may comprise a plurality of sensors placed around each ears of the wearer and at least one sensor around the nose of the wearer, each sensor being connected to a smart frame.

As illustrated on figure 2, the invention also relates to a method for communicating between a wearable device and a wearable sensor.

As described previously, the wearable sensor is configured to be worn by a wearer and to measure data relative to the wearer when worn by the wearer, the wearable device comprising a communication component and a power source.

The method comprising at least when the wearable device and the wearable sensor are in contact:
- a data receiving step S2
- an electrical power providing step S4.

During the data receiving step S2, data is received by the communication component from the wearable sensor. As described previously, the data is measured by the wearable sensor and relates to the wearer.

During the electrical power providing step S4, electrical power is provided to the wearable sensor from the power source comprised in the wearable device.

The method of the invention may comprise prior to the data receiving step and electrical power providing step, an optimal couple of connecting plots determining step S1.

During the optimal couple of connecting plots determining step S1, at least two connecting plots of the wearable device, at least two corresponding connecting plots of the wearable sensor each matching with a connecting plot of the wearable device are determined.

The data received by the wearable device from the wearable sensor may be stored in a memory embedded in the wearable device during a data storing step S3.

As described previously, the data received from the wearable sensor may be transmitted from the communication component of the wearable device to a distant unity comprising a storage unit during a data transmitting step S5.

The invention may be used for quantified-self applications or health monitoring.

For example, the invention may be used for applications such as health senior monitoring, sport monitoring, and many special monitoring, like eye movement, fatigue, stress monitoring ...

For example, it is possible to have pollution sensors on the patch, or on one side of a piercing implanted on the nose, and communicate according to the method of the invention with a frame thru the contact points on the nose.

In case of nose piercing a gas sensor can be situated inside the nose and thus give exact information of the quantity of gas or particle absorbed by the wearer, and the other side of the piercing can be connected to a spectacle frame, that comprises at least a communication system and a battery, and optionally a processing unit. Pollution can be organic volatile components, CO₂ or pollen. Many gas and pollution sensors are already available, many of them are small enough to be embedded into a skin patch or a piercing.

A similar system can also be used to analyze the breathing of the wearer such as the quantity and speed of air inhaled.

As transparent skin patch sensors exist, it is possible to have muscle activity sensor on different parts of the face of the wearer and connection to a spectacle frame thru the ear or nose. Such sensors can be used to detect eyelid, eye movement, emotion, or other muscle activity like chewing muscles to monitor people who need to control their eating habit, like children who does not chew enough or elderly people who do not eat at regular period.

Movement sensors can also be used on the nose to monitor breathing activity during sleep. Other movement sensors can help to analyze sleep quality, and detect the different sleeping phase by face movement analysis, and rapid eye movement detection.

The invention has been described above with the aid of embodiments without limitation of the general inventive concept.

Many further modifications and variations will suggest themselves to those skilled in the art upon making reference to the foregoing illustrative embodiments, which are given by way of example only and which are not intended to limit the scope of the invention, that being determined solely by the appended claims.
In the claims, the word "comprising" does not exclude other elements or steps, and the indefinite article "a" or "an" does not exclude a plurality. The mere fact that different features are recited in mutually different dependent claims does not indicate that a combination of these features cannot be advantageously used. Any reference signs in the claims should not be construed as limiting the scope of the invention.

## Claims

1. A system comprising a wearable device and a wearable sensor, each one being configured to be in communication with the other one when the wearable device and the wearable sensor are in contact, the wearable sensor when worn by a wearer is configured to measure data relative to the wearer, and the wearable device comprises at least:
- a communication component configured to receive data from the wearable sensor when the wearable sensor is in contact with the wearable device, and
- a power source for providing electrical power to the wearable sensor when the wearable sensor is in contact with the wearable device.

2. System according to claim 1, wherein the wearable device is a wristband and/or a watch and/or a head mounted device.

3. System according to claim 1 or 2, wherein the wearable sensor is a sensor configured to be attached to the body of the wearer.

4. System according to any of the preceding claims 1 to 3, wherein the wearable sensor is a patch configured to be glued on the body of the wearer and/or an implant comprising a connector configured to be glued on the body of the wearer and/or a piercing configured to be attached to the body of the wearer.

5. System according to any of the preceding claims 1 to 4, wherein the wearable device and the wearable sensor are connected with a removable wire.

6. System according to any of the preceding claims 1 to 5, wherein the wearable device and the wearable sensor comprise each at least two connecting plots configured to be in contact when the wearable device and the wearable sensor are in communication.

7. System according to claim 6, wherein each of the wearable device and of the wearable sensor comprises a plurality of connecting plots and wherein the computer executable instructions comprises instructions for determining for at least two connecting plots of the wearable device, two corresponding connecting plots of the wearable sensor each matching with a connecting plot of the wearable device.

8. System according to any of the preceding claims 1 to 7, wherein the wearable device comprises a memory configured to store data received from the wearable sensor.

9. System according to any of the preceding claims 1 to 8, wherein the communication component of the wearable device is configured to transmit data received from the wearable sensor to a distant unity comprising a storage unit.

10. A method for communicating between a wearable device and a wearable sensor, the wearable sensor being configured to be worn by a wearer and to measure data relative to the wearer when worn by the wearer, the wearable device comprising a communication component and a power source, the method comprising at least when the wearable device and the wearable sensor are in contact:
- a data receiving step, during which data is received by the communication component from the wearable sensor, and
- an electrical power providing step, during which electrical power is provided to the wearable sensor from the power source.

11. Method according to claim 10, further comprising a data storing step, during which data received from the wearable sensor are stored in a memory embedded in the wearable device.

12. Method according to claim 10 or 11, wherein each of the wearable device and of the wearable sensor comprises a plurality of connecting plots and wherein the method further comprises an optimal couple of connecting plots determining step, during which for at least two connecting plots of the wearable device, at least two corresponding connecting plots of the wearable sensor each matching with a connecting plot of the wearable device are determined.

13. Method according to any of the preceding claims 10 to 12, further comprising a data transmitting step, during which data received from the wearable sensor are transmitted from the communication component of the wearable device to a distant unity comprising a storage unit.

14. A computer program product comprising one or more stored sequences of instructions that are accessible to a processor and which, when executed by the processor, causes the processor to carry out the steps of the method according to any of the claims 10 to 13.

15. A computer-readable storage medium having a program recorded thereon, where the program makes the computer execute the method according to any of the claims 10 to 13.
